(19) **Europäisches Patentamt**

**European Patent Office**

**Office européen des brevets**

(11) **EP 1 125 574 B1**

(12) **EUROPÄISCHE PATENTSCHRIFT**

(45) Veröffentlichungstag und Bekanntmachung des Hinweises auf die Patenterteilung:
**22.06.2005 Patentblatt 2005/25**

(51) Int Cl.⁷: **A61K 7/48**

(21) Anmeldenummer: **01101895.9**

(22) Anmeldetag: **27.01.2001**

(54) **Kosmetische und pharmazeutische Öl-in-Wasser-Emulsionen von polyethermodifizierten Polysiloxanen**

Cosmetical and pharmaceutical oil-in-water emulsions of polyether polysiloxanes

Emulsions cosmétiques et pharmaceutiques du type huile-dans-l'eau contenant des polyéther polysiloxanes

(84) Benannte Vertragsstaaten:
**AT BE CH CY DE DK ES FI FR GB GR IE IT LI LU MC NL PT SE TR**

(30) Priorität: **19.02.2000 DE 10007649**

(43) Veröffentlichungstag der Anmeldung:
**22.08.2001 Patentblatt 2001/34**

(73) Patentinhaber: **Goldschmidt GmbH**
**45127 Essen (DE)**

(72) Erfinder:
• **Dietz, Thomas, Dr.**
  **45259 Essen (DE)**
• **Hameyer, Peter**
  **45138 Essen (DE)**
• **Jenni, Klaus, Dr.**
  **45357 Essen (DE)**

(56) Entgegenhaltungen:
**EP-A- 0 279 319**          **EP-A- 0 819 426**
**DE-A- 2 829 388**          **US-A- 5 132 047**

**Beschreibung**

Die Erfindung betrifft die Verwendung von polyethermodifizierten Polysiloxanen definierter Struktur zur Herstellung kosmetischer und pharmazeutischer Öl-in-Wasser-Emulsionen sowie Öl-in-Wasser-Emulsionen, die diese enthalten.

Der überwiegende Teil kosmetischer und pharmazeutischer Emulsionen ist vom Typ Öl-in-Wasser, d. h. die Ölphase ("disperse Phase") ist in Form kleiner Tröpfchen in der Wasserphase ("kohärente Phase") feinst verteilt. Die Viskosität von Emulsionen, die nur aus Wasser, Öl und Emulgator bestehen und deren Gehalt an disperser Phase unter 60 Gew.-% liegt, ist gleich der Viskosität der kohärenten Phase, im Fall von Öl-in-Wasser-Emulsionen also gleich der von Wasser. Kosmetische Emulsionen enthalten aus Gründen des Hautgefühls im Durchschnitt nicht mehr als 30 % Ölphase, d. h. auch sie wären *per se* wasserdünn. Da aber vom Verbraucher in der Regel lotionsartige (dickflüssige) bis cremeartige (halbfeste) Konsistenz gewünscht wird, und auch die Stabilität von Emulsionen mit der Viskosität der kohärenten Phase zunimmt, ist das "Verdicken" von Öl-in-Wasser-Emulsionen unerlässlich. Dazu gibt es zwei grundsätzlich unterschiedliche Methoden, die auch miteinander kombiniert werden können. Die eine Methode beruht darauf, dass bestimmte Öl-in-Wasser-Emulgatoren dazu in der Lage sind, zusammen mit sogenannten "hydrophilen Wachsen" flüssigkristalline (lamellare) Strukturen in der kohärenten Wasserphase auszubilden, die sich zu einem dreidimensionalen Netzwerk zusammenfügen, das zum einen zu einer starken Viskositätserhöhung der Emulsion führt und zum anderen die Öltröpfchen voneinander getrennt hält und so die Stabilität der Emulsion verbessert. Beispiele für "hydrophile Wachse" sind Stearylalkohol, Stearinsäure und Glycerylstearat. Die andere Methode beruht auf der Fähigkeit von sogenannten "Hydrokolloiden", ein Vielfaches ihres Eigengewichts an Wasser aufzunehmen und zu binden und so zu einer Verdickung von Wasser zu führen. Beispiele für solche wasserquellbaren Organopolymere sind vernetzte Polyacrylate ("Carbomere") und Polysaccharide, beispielsweise Xanthan-Gum. Nachteil dieser beiden Verdickungsmethoden ist jedoch, dass die dabei verwendeten Substanzen sich während oder nach der Anwendung der Emulsionen negativ auf das Hautgefühl auswirken können. So lassen sich beispielsweise in Anwesenheit größerer Mengen hydrophiler Wachse die Emulsionen nur schwer verteilen und es bleibt oft ein stumpfes, wachsartiges Hautgefühl zurück. Auf der anderen Seite zeigen auch die wasserquellbaren Organopolymere Nachteile in den Applikationseigenschaften. So beobachtet man beispielsweise bei Carbomeren den sogenannten "Quick-Breaking-Effekt". Darunter versteht man das Phänomen, dass bei Kontakt der Emulsion mit den Elektrolyten der Haut die Emulsion sofort bricht, was sich durch ein "wässriges Wegrutschen" beim Einreiben bemerkbar macht und oft als unangenehm empfunden wird.

Zur Herstellung von Öl-in-Wasser-Emulsionen werden üblicherweise Emulgatoren eingesetzt, deren HLB-Wert zwischen 8 und 18 liegt. Der HLB-Wert ist eine dimensionslose Größe zur Charakterisierung von Tensiden und beschreibt das Verhältnis von hydrophilem zu lipophilem Anteil im Molekül (HLB = Hydrophile-Lipophile Balance). So wurde auf Basis zahlreicher Experimente von Griffin (J. Soc. Cosmet. Chem. 1949, 1, 311) gefunden, dass beispielsweise Tenside mit einem HLB-Wert von 3 bis 6 als Wasser-in-Öl-Emulgatoren, mit einem HLB-Wert von 6 bis 8 als Netzmittel und Tenside mit einem HLB-Wert größer als 8 als Öl-in-Wasser-Emulgatoren geeignet sind. Im einfachsten Fall errechnet sich der HLB-Wert aus dem prozentualen Anteil des hydrophilen Teils eines Emulgators, beispielsweise des Polyethylenglycolteils, indem man diesen durch 5 dividiert. So beträgt beispielsweise der hydrophile Anteil im Anlagerungsprodukt von 20 Mol Ethylenoxid (MG = 880 g/mol) an Stearinsäure (284 g/mol) 76 %, entsprechend einem HLB-Wert von 15 (= 76/5). Dieses HLB-Konzept ist ursprünglich auf nichtionogene, keine anderen Atome als Kohlenstoff, Wasserstoff und Sauerstoff enthaltende Substanzen beschränkt. Ferner gilt diese HLB-Wert-Definition nicht exakt für Substanzen, deren hydrophiler Teil neben Ethylenglycol- auch Propylenglycoleinheiten enthält.

Ein Nachteil von Emulgatoren mit einem HLB-Wert von deutlich größer als 8 ist, dass sie weniger mild sind als Emulgatoren mit einem niedrigeren HLB-Wert. Ferner sind sie aufgrund ihrer höheren Hydrophilie leichter re-emulgierbar, das heißt sie lassen sich leichter wieder von der Haut mit Wasser abwaschen, was beispielsweise bei Sonnenschutzformulierungen unerwünscht ist, die wasserfest sein sollen. Umgekehrt bilden Emulgatoren mit einem HLB-Wert um 8 und darunter einen hydrophoben Film auf der Haut, der diese vor übermäßigem Wasserverlust schützt und so einen pflegenden Effekt besitzt. Dies ist wohl der Hauptgrund dafür, dass Wasser-in-Öl-Emulsionen, die Emulgatoren mit einem HLB-Wert von unter 8 erfordern, einen stärkeren Pflegeeffekt besitzen als Öl-in-Wasser-Emulsionen, die eher hydrophile Emulgatoren enthalten. Öl-in-Wasser-Emulsionen werden jedoch in der Regel vom Verbraucher bevorzugt, da sie sich aufgrund der wässrigen äußeren Phase leichter verteilen lassen.

Öl-in-Wasser-Emulsionen, die Polyethersiloxane enthalten, sind aus dem Stand der Technik bekannt, wie im folgenden dargelegt wird.

EP 0 154 837 A2 beschreibt niedrigviskose Öl-in-Wasser-Emulsionen mit einer Kombination aus einem kammartigen, endverschlossenen Polyethersiloxan, einem Tensid mit einem HLB-Wert nicht kleiner als 10 und einem Fettalkohol als Emulgatoren, die einen niedrigen Ölphasengehalt besitzen und deren Ölphase überwiegend aus Siliconöl besteht und zusätzlich die Wasserphase Ethanol enthält.

EP 0 279 319 A beschreibt pigmenthaltige Öl-in-Wasser-Emulsionen mit einem Polyethersiloxan als Emulgator, dessen Polyetherrest maximal 50 Mol-% Polyoxypropylen-Einheiten enthält, und deren Ölphase überwiegend aus unmodifizierten oder alkylmodifizierten Siliconölen besteht.

EP 0 516 547 A beschreibt Öl-in-Wasser-Emulsionen mit einem kammartigen Polyethersiloxan mit einem HLB-Wert von 9 bis 12 als Emulgator, dessen Polyether ausschließlich aus Polyethylenoxid mit endständiger OH-Gruppe besteht. Die Ölphase besteht aus einem kettenförmigen oder einem cyclischen Siloxan.

DE 42 41 799 C1 beschreibt kosmetische Mittel, die in zwei separaten, optisch voneinander getrennten Phasen vorliegen, durch Schütteln unmittelbar vor der Anwendung zu einer homogenen Emulsion vereinigt werden können und nach erfolgter Anwendung sich rasch wieder in separate Phasen trennen. Als Emulgator wird ein kammartiges Polyethersiloxan verwendet.

Aus EP 0 627 259 A2 ist bekannt, dass auch mit Siliconpolyethern mit einem HLB-Wert zwischen 4 und 7 Siliconin-Wasser-Emulsionen erhalten werden können. Diese werden durch Einrühren einer Ölphase, die aus Siliconöl und einem ersten Siliconpolyether besteht, in eine Wasserphase, die einen zweiten Siliconpolyether enthält, hergestellt. Beide Siliconpolyether sind kammartig aufgebaut.

Aufgabe der EP 0 819 426 A war die Bereitstellung von Emulgatoren für Antiperspirantformulierungen auf der Basis von W/O-Emulsionen, die eine niedrige Viskosität aufweisen und damit ohne den Einsatz von Lösungsmitteln oder Dispersionen handhabbar sind und die gewünschte emulgierende Wirkung in möglichst geringer Konzentration entfalten.

Gelöst wird diese Aufgabe durch hydrophobe Polyethersiloxane mit zwei endständigen Polyetherketten, wobei die Ölphase in wesentlichen Mengen aus Siliconölen besteht.

Der Stand der Technik lässt sich so zusammenfassen, dass Öl-in-Wasser-Emulsionen mit Siliconpolyethern als Emulgatoren bekannt sind, bei denen die Ölphase zum überwiegenden Teil aus Siliconölen besteht und der Siliconpolyether kammartig aufgebaut ist.

Gegenstand der Erfindung ist in einer ersten Ausführungsform eine kosmetische oder pharmazeutische Öl-in-Wasser-Emulsion, die ein oder mehrere Polyethersiloxane der allgemeinen Formel (I)

$$R(CH_3)_2SiO\text{-}[(CH_3)_2SiO]_n\text{-}Si(CH_3)_2R \qquad (I)$$

wobei
n = 50 bis 250

$$R = \text{-}(CH_2)_m\text{-}O\text{-}(C_2H_4O)_x\text{-}(C_3H_6O)_yR^1$$

m = 2 bis 4
x = 3 bis 100
y = 0 bis 50
$R^1$ = H, $CH_3$, oder $CH_2CH_3$
ist,
mit einem Gewichtsanteil der Polyetherreste R von bis zu 45 Gew.-% an der Gesamtmolekularmasse, berechnet nach Formel (II)

$$\text{"Gewichtsanteil (in \%) der Polyetherreste R an der}$$

$$\text{Gesamtmolekularmasse"} = (MG_{Polyetherreste}/MG_{Gesamt})\cdot100 \qquad (II)$$

mit

$$MG_{Gesamt} = MG_{Siliconrest} + MG_{Polyetherreste}$$

$$MG_{Siliconrest} = n \cdot 74{,}1 + 132{,}2$$

$$MG_{Polyetherreste} = 2 \cdot (m \cdot 14 + 16 + x \cdot 44 + y \cdot 58 + z)$$

mit z = 1, 15 oder 29,

enthält.

Eine weitere Ausführungsform der Erfindung umfaßt kosmetische oder pharmazeutische Öl-in-Wasser-Emulsionen, enthaltend

(a) ein oder mehrere Polyethersiloxane der allgemeinen Formel (I)
(b) flüssig-kristalline Strukturen ausbildende hydrophile Wachse und/oder wasserquellbare Organopolymere als Konsistenzgeber und Stabilisatoren
(c) kosmetische Öle und Wachse und
(d) übliche Hilfs- und Wirkstoffe.

Es wurde überraschend gefunden, dass mit eher hydrophoben polyethermodifizierten Polysiloxanen definierter Struktur als emulgieraktive Komponente homogene und stabile Öl-in-Wasser-Emulsionen erhalten werden können, insbesondere auch Öl-in-Wasser-Emulsionen, die wenig oder gar keine Siliconverbindungen als Ölkomponenten enthalten. Ferner war überraschend, dass mit diesem speziellen Typ von Polyethersiloxanen die in kosmetischen Öl-in-Wasser-Emulsionen üblichen konsistenzgebenden Strukturen, seien es die flüssig-kristallinen Strukturen der hydrophilen Wachse oder die aus wasserquellbaren Organopolymeren gebildeten Gelstrukturen, weniger gestört werden als mit üblichen hydrophilen Polyethersiloxanen. Diese Störung macht sich beispielsweise durch ein grießiges Aussehen unmittelbar nach Herstellung einer Emulsion bemerkbar, deren cremeartige Konsistenz mit Hilfe von hydrophilen Wachsen erzeugt wurde oder, im Fall von wasserquellbaren Organopolymeren, durch eine niedrigere Viskosität der Emulsion. Außerdem war nicht vorhersehbar, dass die erfindungsgemäß eingesetzten Polyethersiloxane die durch die üblichen Konsistenzgeber verursachten Nachteile in den Applikationseigenschaften, wie beispielsweise das stumpfigwachsige Hautgefühl, der "Quick-Breaking-Effekt" oder das "Weißeln" (= Schäumen beim Einreiben), minimieren oder sogar gänzlich aufheben und selbst direkt das Hautgefühl positiv beeinflussen. Die Haut fühlt sich, insbesondere nach dem Einreiben der Emulsion ("After Feel"), samtig-seidig und außerordentlich glatt an, was zusätzlich auch noch lange anhält. Dieses einzigartige Hautgefühl wird nicht mit marktüblichen organischen Emulgatoren oder anderen als den erfindungsgemäß verwendeten $\alpha,\omega$-Polyethersiloxanen, auch nicht in Kombination mit öllöslichen Siliconverbindungen wie beispielsweise cyclischen oder kettenförmigen Polydimethylsiloxanen, erzielt. Eine besondere Ausführungsform dieser Erfindung umfaßt daher Öl-in-Wasser-Emulsionen, die frei von siliconartigen Ölkomponenten sind.

Aufgrund des eher hydrophoben Charakters dieser Polyethersiloxane ist ferner zu erwarten, dass sie besonders hautmild sind, einen hydrophoben Film auf der Haut bilden, der die Haut vor dem Austrocknen schützt und selbst nur schwer mit Wasser wieder entfernbar ist, was zum Beispiel für wasserfeste Sonnenschutzpräparate nützlich ist.

Aus früheren Arbeiten des Standes der Technik ist bekannt, dass Polyethersiloxane gleich welcher Art allein ohne Co-Emulgator nicht in der Lage sind, im Wechselspiel mit hydrophilen Wachsen wie Stearylalkohol oder Glycerylstearat flüssig-kristalline Strukturen in der kohärenten Wasserphase aufzubauen und damit die erforderliche lotionsoder cremeartige Konsistenz sowie Stabilität zu liefern. Es war jedoch überraschend, dass dies bereits mit nur einem geringen Anteil eines Co-Emulgators möglich ist und dass homogene und langzeitstabile Emulsionen nur mit den erfindungsgemäß verwendeten Polyethersiloxanen erhalten werden können. In einem Vergleichsexperiment mit beispielsweise einem kammartigen, hydrophilen Polysiloxan war die Creme nach Herstellung und Abkühlen stark inhomogen und grießig.

Zur genauen Abgrenzung der erfindungsgemäß eingesetzten Polyethersiloxane zu den aus dem Stand der Technik bekannten, zur Herstellung von Öl-in-Wasser-Emulsionen verwendeten Polyethersiloxanen wird auf die Angabe eines HLB-Wertes zugunsten einer Angabe des Gewichtsanteils der Polyetherreste am Gesamtmolekulargewicht verzichtet, zumal eine klassische Berechnung des HLB-Werts nicht korrekt wäre, da diese Klasse von Emulgatoren Silicium-Atome enthalten und zusätzlich im Polyetherrest auch Propylenglycoleinheiten zugelassen sind. Auch eine Charakterisierung mit Hilfe des sogenannten "dreidimensionalen HLB-Konzepts" von A. J. O'Lenick et al. (Cosm. & Toil., 111, 1996, 37 - 44) erscheint wenig sinnvoll. Denn dieses System sagt voraus, dass mit Siliconpolyethern keine stabilen Öl-in-Wasser- oder Wasserin-Öl-Emulsionen erhalten werden können, da Siliconpolyether keine Komponenten enthalten, die in einer rein organischen Ölphase löslich sind. Genau dies wird mit der erfindungsgemäßen Verwendung der im folgenden beschriebenen Polyethersiloxane widerlegt. Beim "dreidimensionalen HLB-Konzept" wird zusätzlich zur wasser- und öllöslichen Komponente eines Emulgators auch eine siliconlösliche Komponente berücksichtigt. Ein Emulgator wird so durch einen HLB-Wert für den wasserlöslichen Anteil (0 - 20) und einem HLB-Wert für den öllöslichen Anteil (0 - 20) bereits eindeutig charakterisiert, wobei sich der HLB-Wert für den siliconlöslichen Anteil aus der Differenz von 20 und der Summe der HLB-Werte für den wasser- und öllöslichen Anteil ergibt. In einem rechtwinkligen Dreieck, dessen Hypotenuse die klassische HLB-Skala von 0 bis 20 darstellt, sind die Bereiche mit den entsprechenden HLB-Werten des Emulgators eingegrenzt, in denen stabile Emulsionen eines bestimmten Typs erhalten werden. Mögliche Emulsionstypen sind Wasserin-Öl, Öl-in-Wasser, Wasser-in-Silicon, Silicon-in-Wasser, Öl-in-Silicon und Silicon-in-Öl. Aus dem HLB-Dreieck ist so beispielsweise ersichtlich, dass Siliconpolyether (der HLB-Wert für den öllöslichen Anteil ist in diesem Fall 0) mit einem HLB-Wert von 9 bis 18 für den wasserlöslichen Anteil Silicon-in-Wasser-Emulsionen ergeben, während mit einem HLB-Wert von 3 bis 6 Wasser-in-Silicon-Emulsionen. Es kann ferner daraus abgeleitet werden, dass mit Siliconpolyethern keine stabilen Öl-in-Wasser- oder Wasser-in-Öl-Emulsionen erhalten werden sollten. Dies scheint naheliegend, da Siliconpolyether keine Komponenten enthalten, die in einer organischen Ölphase löslich sind. Dies erklärt auch, warum bislang im Stand der Technik nur Emulsionen mit Siliconpolyethern beschrieben werden, die ausschließlich oder überwiegend Siliconöl als zweite Phase neben der Wasserphase enthalten.

Die erfindungsgemäß eingesetzten Polyethersiloxane zeichnen sich dadurch aus, dass sie im Gegensatz zu den im Stand der Technik benutzten Siliconpolyethern nicht kammartig, sondern an den beiden Enden der linearen unverzweigten Siliconkette die Polyetherreste tragen und der Gewichtsanteil der Polyetherreste an der Gesamtmolekularmasse kleiner oder gleich 45 % ist. Die erfindungsgemäßen Emulsionen können ferner zusätzlich einen oder mehrere Co-Emulgatoren, jedoch in geringerem Anteil als die erfindungsgemäß verwendeten Polyethersiloxane, sowie für kosmetische Emulsionen typische Konsistenzgeber und Stabilisatoren enthalten.

In einer weiteren Ausführungsform umfaßt die Erfindung Emulsionen, die Polyethersiloxane der allgemeinen Formel (I) in Kombination mit weiteren Emulgatoren enthalten, wobei jedoch der Anteil der Polyethersiloxane der allgemeinen Formel (I) an der Summe der Emulgatoren mehr als 50 Gew.-%, vorzugsweise 65 bis 90 Gew.-% beträgt.

Als weitere Emulgatoren kommen beispielsweise nichtionogene Tenside aus mindestens einer der folgenden Gruppen in Frage:

- Kammartige Polyethersiloxane

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe

- C12/18-Fettsäuremono- und -diester von Anlagerungsprodukten von 1 bis 30 Mol Ethylenoxid an Glycerin

- Glycerinmono- und -diester und Sorbitanmono- und -diester von gesättigten und ungesättigten Fettsäuren mit 6 bis 22 Kohlenstoffatomen und deren Ethylenoxidanlagerungsprodukte

- Alkylmono- und -oligoglycoside mit 8 bis 22 Kohlenstoffatomen im Alkylrest und deren ethoxylierte Analoga

- Anlagerungsprodukte von 15 bis 60 Mol Ethylenoxid an Ricinusöl und/oder gehärtetes Ricinusöl

- Polyol- und insbesondere Polyglycerinester, wie beispielsweise Polyglycerinpolyricinoleat, Polyglycerin-12-hydroxystearat oder Polyglycerindimerat. Ebenfalls geeignet sind Gemische von Verbindungen aus mehreren dieser Substanzklassen

- Anlagerungsprodukte von 2 bis 15 Mol Ethylenoxid an Ricinusöl und/oder gehärtetes Ricinusöl

- Partialester auf Basis linearer, verzweigter, ungesättigter bzw. gesättigter C6/22-Fettsäuren, Ricinolsäure sowie 12-Hydroxystearinsäure und Glycerin, Polyglycerin, Pentaerythrit, Dipentaerythrit, Zuckeralkohole (beispielsweise Sorbit), Alkylglucoside (beispielsweise Methylglucosid, Butylglucosid, Laurylglucosid) sowie Polyglucoside (beispielsweise Cellulose)

- Mono-, Di- und Trialkylphosphate sowie Mono-, Di- und/oder Tri-PEG-alkylphosphate und deren Salze

- Wollwachsalkohole

- Polysiloxan-Polyalkyl-Polyether-Copolymere beziehungsweise entsprechende Derivate

- Mischester aus Pentaerythrit, Fettsäuren, Citronensäure und Fettalkohol gemäß DE-PS 11 65 574 und/oder Mischester von Fettsäuren mit 6 bis 22 Kohlenstoffatomen, Methylglucose und Polyolen, vorzugsweise Glycerin oder Polyglycerin sowie

- Polyalkylenglycole

- Betaine

- Esterquats

- Natrium-, Kalium- oder Ammoniumsalze von langkettigen Alkyl- und Alkylethersulfonsäuren.

Die Anlagerungsprodukte von Ethylenoxid und/oder von Propylenoxid an Fettalkohole, Fettsäuren, Alkylphenole, Glycerinmono- und diester sowie Scrbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Cocosalkyldimethylammoniumglycinat, N-Acylaminopropyl-N,N-dimethylammoniumglycinate, beispielsweise das Cocosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Cocosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C8/18-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Cocosalkylaminopropionat, das Cocosacylaminoethylaminopropionat und das C12/18-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Eine weitere Ausführungsform der erfindungsgemäßen Öl-in-Wasser-Emulsionen umfassen solche, die hydrophile Wachse ausgewählt aus der Gruppe, bestehend aus Stearylalkohol, Stearinsäure und/oder Glycerylstearat als Konsistenzgeber sowie als Co-Emulgator einen organischen Emulgator enthalten, der in der Lage ist, zusammen mit den hydrophilen Wachsen flüssigkristalline Strukturen aufzubauen. Bevorzugt ist ein Anteil von 5 bis 49 Gew.-% des organischen Co-Emulgators an der Gesamtmenge der Emulgatoren, besonders bevorzugt ein Anteil von 10 bis 35 Gew.-%. Der Anteil des erfindungsgemäß verwendeten Polyethersiloxans beträgt mindestens 51 Gew.-% an der Gesamtmenge der Emulgatoren.

Als Konsistenzgeber kommen in erster Linie Fettalkohole oder Hydroxyfettalkholole mit 12 bis 22 und vorzugsweise 16 bis 18 Kohlenstoffatomen und daneben Partialglyceride, Fettsäuren oder Hydroxyfettsäuren in Betracht. Geeignete Verdickungsmittel sind beispielsweise Polysaccharide, insbesondere Xanthan-Gum, Guar-Guar, Agar-Agar, Alginate und Tylosen, Carboxymethyl-cellulose und Hydroxyethylcellulose, ferner höhermolekulare Polyethylenglycolmono- und diester von Fettsäuren, Polyacrylate (beispielsweise Carbopole von Goodrich, TEGO Carbomere von Goldschmidt oder Synthalene von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside.

Als Ölphase kommen beispielsweise solche Ölkomponenten in Frage, die als kosmetische und pharmazeutische Ölkomponenten sowie als Gleit- und Schmiermittelkomponenten bekannt sind. Hierzu zählen insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen. Ebenso sind im erfindungsgemäßen Sinne die Veresterungsprodukte aliphatischer, difunktioneller Alkohole mit 2 bis 36 C-Atomen mit monofunktionellen aliphatischen Carbonsäuren mit 1 bis 22 C-Atomen geeignet. Als Ölkomponenten geeignete Monoester sind beispielsweise die Methylester und Isopropylester von Fettsäuren mit 12 bis 22 C-Atomen wie beispielsweise Methyllaurat, Methylstearat, Methyloleat, Methylerucat, Isopropylpalmitat, Isopropylmyristat, Isopropylstearat, Isopropyloleat in Frage. Andere geeignete Monoester sind beispielsweise n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, beispielsweise Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie beispielsweise im Jojobaöl oder im Spermöl vorliegen.

Geeignete Dicarbonsäureester sind beispielsweise Di-n-butyladipat, Di-n-butyl-sebacat, Di-(2-ethylhexyl)-adipat, Di-(2-hexyldecyl)-succinat, D-isotridecylacelaat. Geeignete Diolester sind beispielsweise Ethylenglycoldioleat, Ethylenglycoldi-isotridecanoat, Propylenglycol-di-(2-ethylhexanoat), Butandiol-di-isostearat und Neopentylglycol-di-caprylat.

Ebenso als Ölkomponente geeignet sind die Fettsäuretriglyceride, wobei unter diesen die natürlich vorkommenden Öle und Fette bevorzugt sind. So kommen beispielsweise natürliche, pflanzliche Öle, beispielsweise Olivenöl, Sonnenblumenöl, Sojaöl, Erdnussöl, Rapsöl, Mandelöl, Palmöl aber auch die flüssigen Anteil des Kokosöls oder des Palmkernöls sowie tierische Öle wie beispielsweise Klauenöl, die flüssigen Anteile des Rindertalgs oder auch synthetische Triglyceride von Capryl-Caprinsäure-Gemischen, Triglyceride aus technischer Ölsäure oder aus Palmitinsäure-Ölsäure-Gemischen als Ölkomponenten in Frage.

Als weitere Hilfs- und Zusatzstoffe kommen unter anderem UV-Lichtschutzfilter in Frage.

Unter UV-Lichtschutzfiltern sind organische Substanzen zu verstehen, die in der Lage sind, ultraviolette Strahlen zu absorbieren und die aufgenommene Energie in Form längerwelliger Strahlung, beispielsweise Wärme wieder abzugeben. UVB-Filter können öllöslich oder wasserlöslich sein. Als öllösliche Substanzen sind beispielsweise zu nennen:

- 3-Benzylidencampher und dessen Derivate, beispielsweise 3-(4-Methylbenzyliden)camphor

- 4-Aminobenzoesäurederivate, vorzugsweise 4-(Dimethylamino)-benzoesäure-2-ethylhexylester, 4-(Dimethylamino)benzoesäure-2-ethylhexylester und 4-(Dimethylamino)benzoesäureamylester

- Ester der Zimtsäure, vorzugsweise 4-Methoxyzimtsäure-2-ethylhexylester, 4-Methoxyzimtsäureisopentylester, 2-Cyan-3-phenyl-zimtsäure-2-ethylhexylester (Octocrylene)

- Ester der Salicylsäure, vorzugsweise Salicylsäure-2-ethylhexylester, Salicylsäure-4-isopropylbenzylester, Salicylsäurehomomenthylester

- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon

- Ester der Benzalmalonsäure, vorzugsweise 4-Methoxybenzalmalonsäuredi-2-ethylhexylester

- Triazinderivate, wie beispielsweise 2,4,6-Trianilino-(p-carbo-2'-ethyl-1'-hexyloxy)-1,3,5-triazin und Octyltriazon.

- Propan-1,3-dione, wie beispielsweise 1-(4-tert.Butylphenyl)-3-(4'-methoxyphenyl)propan-1,3-dion.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze

- Sulfonsäurederivate von Benzophenon, vorzugsweise 2-Hydroxy-4-methoxybenzophenon-5-sulfonsäure und ihre Salze

- Sulfonsäurederivate des 3-Benzylidencamphers, wie beispielsweise 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.-Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden. Neben den genannten löslichen Stoffen kommen für diesen Zweck auch unlösliche Pigmente, nämlich feindisperse Metalloxide beziehungsweise Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazol-2-yl)-4-(1,1,3,3-tetramethylbutyl)-phenol} mit einer Partikelgröße von kleiner 200 nm, das beispielsweise als 50%ige wässrige Dispersion erhältlich ist.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (beispielsweise Glycin, Histidin, Tyrosin, Tryptophan) und deren Derivate, Imidazole (beispielsweise Urocaninsäure) und deren Derivate, Peptide wie D,L-Carnosin, D-Carnosin und deren Derivate (beispielsweise Anserin), Carotinoide, Carotine (beispielsweise α-Carotin, β-Carotin, Lycopin) und deren Derivate, Chlorogensäure und deren Derivate, Liponsäure und deren Derivate (beispielsweise Dihydroliponsäure), Aurothioglucose, Propylthiouracil und andere Thiole (beispielsweise Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiopropionat, Distearylthiopropionat, Thiodipropionsäure und deren Derivate (Ester, Ether, Peptide, Lipide, Nukleotide, Nukleoside und Salze) sowie Sulfoximinverbindungen (beispielsweise Buthioninsulfoximine, Homocysteinsulfoximin, Buthioninsulfone, Penta-, Hexa-, Heptathioninsulfoximin) in sehr geringen verträglichen Dosierungen (beispielsweise pmol bis µmol/kg), ferner (Metall)-Chelatoren (beispielsweise α-Hydroxyfettsäuren, Palmitinsäure, Phytinsäure, Lactoferrin), α-Hydroxysäuren (beispielsweise Citronensäure, Milchsäure, Äpfelsäure), Huminsäure, Gallensäure, Gallenextrakte, Bilirubin, Biliverdin, EDTA, EGTA und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (beispielsweise Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihadroguajaretsäure, Trihydroxybutyrophenon, Harnsäure und deren Derivate, Mannose und deren Derivate, Superoxid-Dismutase, Zink und dessen Derivate (beispielsweise ZnO, $ZnSO_4$),Selen und dessen Derivate (beispielsweise Selen-methionin), Stilbene und deren Derivate (beispielsweise Stilbenoxid, trans-Stilbenoxid) und die erfindungsgemäß geeigneten Derivate (Salze, Ester, Ether, Zucker, Nucleotide, Peptide und Lipide) dieser genannten Wirkstoffe.

Als Konservierungsmittel eigene sich beispielsweise Phenoxyethanol, Formaldehydlösung, Parabene, Pentandiol oder Sorbinsäure.

Als Insekten-Repellentien kommen N,N-Diethyl-m-toluamid, 1,2-Pentandiol oder Insect Repellent 3535 in Frage, als Selbstbräuner eignet sich Dihydroxyaceton, als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycidat, Allylcyclohexylpropionat, Styrallylpropionat und Benzylsalicylat. Zu den Ethern zählen beispielsweise Benzylethylether, zu den Aldehyden beispielsweise die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen beispielsweise die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, beispielsweise Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Als Deodorantwirkstoffe kommen z. B. Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Illit, Beidellit, Nontronit, Saponit, Hectorit, Bentonit, Smectit, ferner beispielsweise Zinksalze der Ricinolsäure in Frage.

Keimhemmende Mittel sind ebenfalls geeignet, in die erfindungsgemäßen Öl-in-Wasser-Emulsionen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-198 55 934, DE-37 40 186, DE-39 38 140, DE-42 04 321, DE-42 29 707, DE-42 29 737, DE-42 38 081, DE-43 09 372, DE-43 24 219 beschriebenen wirksamen Agenzien. Weitere übliche Antitranspirantwirkstoffe können ebenfalls vorteilhaft in den erfindungsgemäßen Zubereitungen verwendet werden, insbesondere Adstringentien, beispielsweise basische Aluminiumchloride, wie Aluminiumchlorhydrat ("ACH") und Aluminium-Zirkonium-Glycine-Salze ("ZAG").

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 - 106 zusammengestellt sind. Diese Farbstoffe werden üblicherweise in Konzentrationen von 0,001 bis 0,1 Gew.-%, bezogen auf die gesamte Mischung, eingesetzt.

Als Wirkstoffe sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe geeignet.

Eine weitere Ausführung der erfindungsgemäßen Öl-in-Wasser-Emulsionen umfassen solche, die frei sind von öllöslichen Siliconverbindungen, insbesondere flüchtigen cyclischen Polydimethylsiloxanen.

Ausführungsbeispiele:

Bezugsbeispiele 1 bis 5:

Beispiele erfindungsgemäß verwendeter Polyethersiloxane der allgemeinen Formel (I) sind in der folgenden Tabelle aufgeführt:

| Beispiel | n | MG$_{Siliconrest}$ | m | x | y | z | MG$_{Polyetherreste}$ | Gew. -Anteil Polyetherreste in [%]* |
|----------|-----|--------|---|----|----|----|------|----|
| 1 | 66 | 5048 | 3 | 13 | 0 | 1 | 1262 | 20 |
| 2 | 50 | 3837 | 3 | 15 | 10 | 15 | 2626 | 41 |
| 3 | 200 | 14952 | 3 | 13 | 20 | 1 | 3582 | 19 |
| 4 | 100 | 7542 | 3 | 11 | 17 | 1 | 3058 | 29 |
| 5 | 150 | 11247 | 3 | 19 | 3 | 29 | 2194 | 16 |

*berechnet nach Formel (II)

Beispiele erfindungsgemäßer Öl-in-Wasser-Emulsionen sind im folgenden aufgeführt:

Beispiel 1:

| A | Polyethersiloxan Bezugsbeispiel 5 | 2,0 % |
|---|-----------------------------------|-------|
|   | Capryl/Caprin-Triglycerid | 10,4 % |
|   | Ethylhexyl-Stearat | 5,0 % |
|   | Mineral-Öl (30 mPas) | 5,0 % |
|   | Tocopheryl-Acetat | 1,0 % |
| B | Glycerin | 2,0 % |
|   | Panthenol | 1,0 % |
|   | Allantoin | 0,1 % |
|   | Alkohol (Ethanol) | 10,0 % |
|   | Wasser | 66,2 % |
| C | TEGO® Carbomer 140 (Carbomer) | 0,15% |
|   | TEGO® Carbomer 141 (Carbomer) | 0,15 % |

(fortgesetzt)

| | | |
|---|---|---|
| | Xanthan-Gum | 0,1 % |
| | Ethylhexyl-Stearat | 1,6 % |
| D | Natriumhydroxid (10 % in Wasser) | 0,7 % |
| | Konservierungsstoff, Parfum | q. s. |

Beispiel 2:

| | | |
|---|---|---|
| A | Polyethersiloxan Bezugsbeispiel 4 | 2,3 % |
| | ABIL® B 8863[1] | 0,3 % |
| | Capryl/Caprin-Triglycerid | 10,4 % |
| | Isohexadecan | 5,0 % |
| B | Wasser | 79,3 % |
| C | TEGO® Carbomer 140 (Carbomer) | 0,3 % |
| | Xanthan-Gum | 0,1 % |
| | Mineral-Öl (30 mPas) | 1,6 % |
| D | Natriumhydroxid (10 % in Wasser) | 0,7 % |
| | Konservierungsstoff, Parfum | q. s. |

[1]ABIL® B 8863: Kammartiges Polyethersiloxan mit Gewichtsanteil der Polyetherreste an der Gesamtmolekularmasse von 76 %.

Beispiel 3:

| | | |
|---|---|---|
| A | Polyethersiloxan Bezugsbeispiel 1 | 2,0 % |
| | C12-15 Alkyl-Benzoat | 3,0 % |
| | Decyl-Cocoat | 2,0 % |
| | Isopropyl-Palmitat | 0,4 % |
| | Avocado-Öl | 1,0 % |
| | 4-Methylbenzyliden-Campher | 3,0 % |
| | Methoxyzimtsäure-ethylhexylester | 2,5 % |
| | p-Methoxyzimtsäure-isoamylester | 2,5 % |
| | Butyl-Methoxydibenzoylmethan | 2,0 % |
| | Tocopheryl-Acetat | 0,5 % |
| B | TEGO® SMO 80 (Polysorbate 80) | 0,2 % |
| | Glycerin | 2,0 % |
| | EDTA | 0,1 % |
| | GluCare® S (Sodium Carboxymethyl Betaglucan) | 0,1 % |
| | Wasser | 75,9 % |
| C | TEGO® Carbomer 140 (Carbomer) | 0,15% |
| | TEGO® Carbomer 141 (Carbomer) | 0,15 % |

(fortgesetzt)

| | | |
|---|---|---|
| | Xanthan-Gum | 0,1 % |
| | Isopropyl-Palmitat | 1,6 % |
| D | Natriumhydroxid (10 % in Wasser) | 0,8 % |
| | Konservierungsstoff, Parfum | q. s. |

Beispiel 4:

| | | |
|---|---|---|
| A | Polyethersiloxan Bezugsbeispiel 2 | 2,0 % |
| | C12-15 Alkyl-Benzoat | 3,0 % |
| | Decyl-Cocoat | 2,0 % |
| | Isopropyl-Palmitat | 0,4 % |
| | Avocado-Öl | 1,0 % |
| | Methoxyzimtsäure-ethylhexylester | 5,0 % |
| | p-Methoxyzimtsäure-isoamylester | 5,0 % |
| | Tocopheryl-Acetat | 0,5 % |
| B | TEGO® SMO 80 (Polysorbate 80) | 0,2 % |
| | Glycerin | 2,0 % |
| | GluCare® S (Beta-Glucan) | 0,1 % |
| | Wasser | 68,6 % |
| C | TEGO® Carbomer 140 (Carbomer) | 0,15% |
| | TEGO® Carbomer 141 (Carbomer) | 0,15 % |
| | Xanthan-Gum | 0,1 % |
| | Isopropyl Palmitat | 1,6 % |
| D | Tinosorb® M (Methylen-bis-benzotriazolyl- tetramethylbutylphenol) (50 %) | 8,0 % |
| E | Natriumhydroxid (10 % in Wasser) | 0,8 % |
| | Konservierungsstoff, Parfum | q. s. |

Beispiel 5:

| | | |
|---|---|---|
| A | Polyethersiloxan Bezugsbeispiel 3 | 1,5 % |
| | TEGINACID® C (Ceteareth-25) | 0,5 % |
| | Stearyl-Alkohol | 2,0 % |
| | Glyceryl-Stearat | 1,0 % |
| | Stearinsäure | 1,0 % |
| | Isopropyl-Palmitat | 5,0 % |
| | Ethylhexyl-Stearat | 5,0 % |
| | Mineral-Öl (30 mPas) | 3,2 % |
| | Tocopheryl-Acetat | 0,3 % |

(fortgesetzt)

| B | Glycerin | 2,0 % |
|---|---|---|
| | Panthenol | 0,5 % |
| | Allantoin | 0,2 % |
| | Wasser | 76,96 % |
| C | TEGO® Carbomer 134 (Carbomer) | 0,1 % |
| | Mineral-Öl (30 mPas) | 0,4 % |
| D | Natriumhydroxid (10 % in Wasser) | 0,25 % |
| | Konservierungsstoff, Parfum | q. s. |

Beispiel 6:

| A | Polyethersiloxan Bezugsbeispiel 1 | 1,5 % |
|---|---|---|
| | PEG-100 Stearat | 0,5 % |
| | Stearyl-Alkohol | 2,0 % |
| | Stearinsäure | 2,0 % |
| | Capryl/Caprin-Triglycerid | 7,0 % |
| | Ethylhexyl-Stearat | 6,2 % |
| | Tocopheryl-Acetat | 0,3 % |
| B | Glycerin | 2,0 % |
| | Panthenol | 0,5 % |
| | Allantoin | 0,2 % |
| | Wasser | 76,96 % |
| C | TEGO® Carbomer 134 (Carbomer) | 0,1 % |
| | Mineral-Öl (30 mPas) | 0,4 % |
| D | Natriumhydroxid (10 % in Wasser) | 0,25 % |
| | Konservierungsstoff, Parfum | q. s. |

Beispiel 7:

| A | Polyethersiloxan Bezugsbeispiel 4 | 1,5 % |
|---|---|---|
| | TEGINACID® C (Ceteareth-25) | 0,5 % |
| | Stearyl-Alkohol | 1,5 % |
| | Glyceryl-Stearat | 2,5 % |
| | Stearyl-Heptanoat | 3,0 % |
| | Cetearyl-Ethylhexanoat | 7,0 % |
| | Decyl-Oleat | 3,5 % |
| B | Glycerin | 3,0 % |
| | Panthenol | 0,5 % |

(fortgesetzt)

| | | | |
|---|---|---|---|
| | | Wasser | 76,16 % |
| C | | TEGO® Carbomer 134 (Carbomer) | 0,1 % |
| | | Mineral-Öl (30 mPas) | 0,4 % |
| D | | Natriumhydroxid (10 % in Wasser) | 0,25 % |
| | | Konservierungsstoff, Parfum | q. s. |

Beispiel 8, Vergleichsbeispiele 1 und 2:

| | Beispiele | Vgl.1 | Vgl.2 | 8 |
|---|---|---|---|---|
| A | Polyethersiloxan Bezugsbeispiel 3 | - | | 1,8 % |
| | ABIL® B 8863[1] | 1,8 % | 1,8 % | - |
| | Ceteareth-25 | - | 0,2 % | 0,2 % |
| | Glyceryl-Stearat | 2,0 % | 2,0 % | 2,0 % |
| | Stearyl-Alkohol | 1,0 % | 1,0 % | 1,0 % |
| | Mineral-Öl | 5,0 % | 5,0 % | 5,0 % |
| | Ethylhexyl-Stearat | 5,0 % | 5,0 % | 5,0 % |
| | Isopropyl-Palmitat | 5,0 % | 5,0 % | 5,0 % |
| B | Glycerin | 2,0 % | 2,0 % | 2,0 % |
| | Wasser | 80,0 % | 80,0 % | 80,0 % |

[1]ABIL® B 8863: Kammartiges Polyethersiloxan mit Gewichtsanteil der Polyetherreste an der Gesamtmolekularmasse von 76 %.

Herstellung: Phase A und Phase B wurden separat auf 70 °C erhitzt, vereinigt und die Mischung 1 min lang intensiv homogenisiert. Anschließend kühlte man im Wasserbad unter Rühren ab. Die Emulsion des Vergleichsbeispiels 1 blieb nach Abkühlen wasserdünn und die Konsistenzgeber lagen als inhomogene Klumpen vor. Die Emulsion des Vergleichsbeispiels 2 wurde zwar cremeartig fest, die Emulsion war jedoch stark inhomogen und grießig, während die erfindungsgemäße Emulsion gemäß Beispiel 8 nach Abkühlen auf Raumtemperatur ein glattes und homogenes Aussehen hatte.

Dieser Vergleich verdeutlicht, dass sich Cremes mit dem erfindungsgemäß eingesetzten Polyethersiloxan gemäß Bezugsbeispiel 3 in Kombination mit dem organischen Co-Emulgator Ceteareth-25 einwandfrei nach dem Heißverfahren herstellen lassen, während sich Cremes mit einer Kombination des Polyethersiloxans ABIL® B 8863 mit Ceteareth-25 nicht herstellen lassen.

Beispiel 9, Vergleichsbeispiele 3 und 4:

| | Beispiele | 9 | Vgl.3 | Vgl. 4 |
|---|---|---|---|---|
| A | Polyethersiloxan Bezugsbeispiel 3 | 1,0 % | | |
| | Hostaphat® KL 340 N (Trilaureth-4 Phosphat) | | 1,0 % | |
| | ABIL® B 8852[1] | | | 1,0 % |
| | MineralÖl | 8,0 % | 8,0 % | 8,0 % |
| | Octyl-Palmitat | 5,0 % | 5,0 % | 5,0 % |

[1]ABIL® B 8852: Kammartiges Polyethersiloxan mit Gewichtsanteil der Polyetherreste an der Gesamtmolekularmasse von 67 %.

(fortgesetzt)

| | Beispiele | | 9 | Vgl.3 | Vgl. 4 |
|---|---|---|---|---|---|
| | | Capryl/Caprin-Triglycerid | 6,0 % | 6,0 % | 6,0 % |
| B | | Glycerin | 2,8 % | 2,8 % | 2,8 % |
| | | Wasser | 75,0 % | 75,0 % | 75,0 % |
| C | | Natriumhydroxid (10 % in Wasser) | 0,7 % | 0,7 % | 0,7 % |
| D | | TEGO® Carbomer 140 (Carbomer) | 0,2 % | 0,2 % | 0,2 % |
| | | Xanthan-Gum | 0,2 % | 0,2 % | 0,2 % |
| | | Octyl-Palmitat | 1,1 % | 1,1 % | 1,1 % |

Herstellung: Phase A wurde gemischt, bis sie homogen war und dann zu Phase B gegeben. Es wurde intensiv homogenisiert. Anschließend gab man Phase C unter leichtem Rühren zu. Schließlich gab man Phase D zu und homogenisierte nochmals kurz.

Die Rezeptur gemäß Beispiel 9 ergab nach Herstellung eine glatte, homogene Emulsion mit einer Viskosität von 9,0 Pas, Rezeptur gemäß Vergleichsbeispiel 3 eine glatte, homogene Emulsion mit einer Viskosität von 4,5 Pas. Rezeptur gemäß Vergleichsbeispiel 4 ergab nach Zugabe der Carbomer/Xanthan Gum-Dispersion eine glasige und inhomogene Emulsion, die bereits nach einigen Minuten separierte.

Dieser Vergleich verdeutlicht, daß die verdickende und stabilisierende Wirkung von Hydrocolloiden wie Carbomeren oder Xanthan Gum von Emulgatoren in unterschiedlicher Weise beeinflußt wird. Von den erfindungsgemäß verwendeten Siliconpolyethern werden sie praktisch nicht in ihrer Wirkung beeinträchtigt; ihre verdickende Wirkung wird durch einen handelsüblichen organischen Emulgator geschwächt, von dem handelsüblichen kammartigen Siliconpolyether ABIL® B 8852 wird dagegen sogar die stabilisierende Wirkung aufgehoben.

Beispiel 10:

In einem Panel-Test wurden 20 Personen gebeten, zwei Körperlotionen bezüglich der Applikationseigenschaften miteinander zu vergleichen. Eine Lotion enthielt 2 % des Polyethersiloxan gemäß Bezugsbeispiel 3 als Emulgator, die andere Lotion einen marktüblichen organischen Emulgator, Eumulgin® VL 75 (Compound aus Lauryl Glucosid, Polyglycerin-2 Dipolyhydroxystearat, Glycerin und Wasser, 4 % entsprechend 2 % emulgieraktive Komponenten); ansonsten waren die Rezepturen identisch.

Ergebnis: Bezüglich der Verteilbarkeit und dem Einziehverhalten wurden die beiden Lotionen praktisch gleich bewertet, das Hautgefühl nach dem vollständigen Einziehen der Lotionen wurde im Fall des Polyethersiloxans jedoch als glatter/weicher und samtiger/seidiger bewertet als im Fall des organischen Emulgators. 17 von 20 Personen würden die Lotion mit dem Polyethersiloxan bevorzugen.

Beispiel 11:

In einem Panel-Test wurden 5 Personen gebeten, zwei Körperlotionen bezüglich der Applikationseigenschaften miteinander zu vergleichen. Eine Lotion enthielt 3 % des Polyethersiloxan gemäß Bezugsbeispiel 5 als Emulgator, die andere Lotion einen marktübliches Polyethersiloxan, ABIL B 8843 (kammartiges Polyethersiloxan mit Gewichtsanteil der Polyetherreste an der Gesamtmolekularmasse von 67 %); ansonsten waren die Rezepturen identisch.

Ergebnis: Bezüglich der Verteilbarkeit und dem Einziehverhalten wurden die beiden Lotionen praktisch gleich bewertet, das Hautgefühl nach dem vollständigen Einziehen der Lotionen wurde im Fall des erfindungsgemäßen Polyethersiloxans jedoch als glatt/weich und samtig/seidig bewertet, während das Hautgefühl im Fall des marktüblichen Polyethersiloxans als trocken und stumpf bewertet wurde. Alle 5 Personen bevorzugten die Lotion mit dem erfindungsgemäßen Polyethersiloxan.

Beispiel 12:

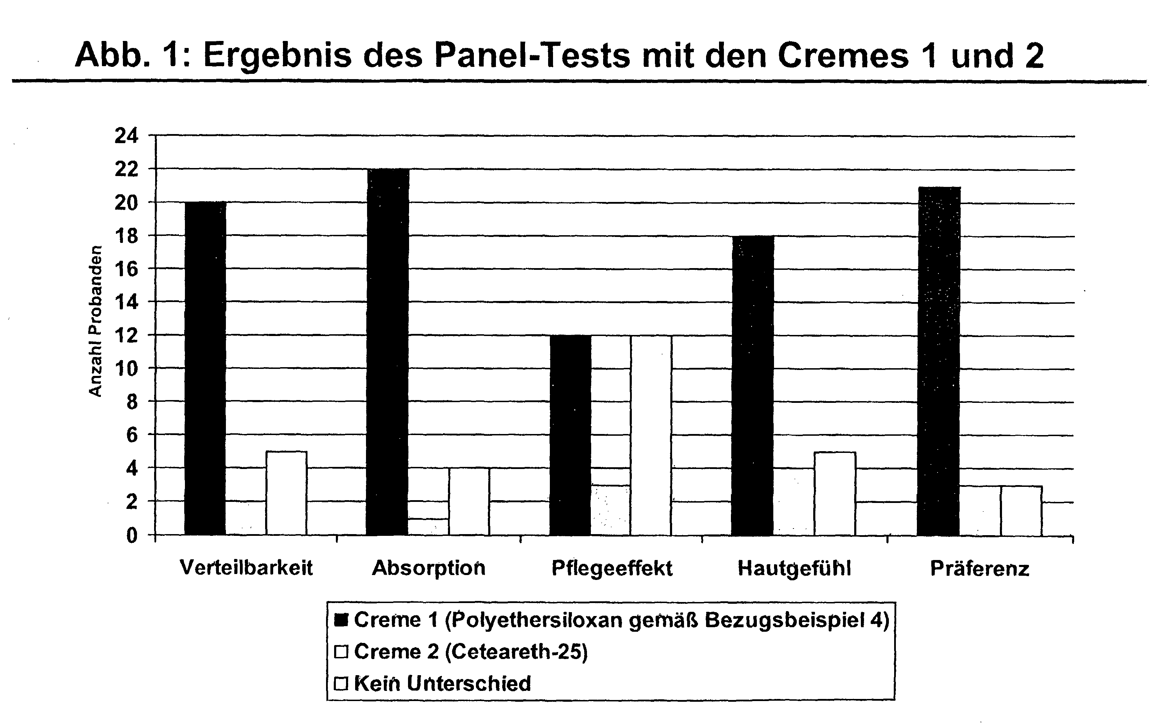

In einem Panel-Test wurden 27 Probanden gebeten, zwei Cremes direkt miteinander zu vergleichen. Creme 1 enthielt eine Kombination aus dem Polyethersiloxan gemäß Bezugsbeispiel 4 und Ceteareth-25 als Co-Emulgator, Creme 2 enthielt ausschließlich Ceteareth-25 als Emulgator (siehe Rezepturen Creme 1 und Creme 2). In Abb. 1 ist das Ergebnis des Paneltests dargestellt: Hinsichtlich der Verteilbarkeit und der Absorption wurde Creme 1 stark bevorzugt, hinsichtlich des Pflegeeffekts gab es eine leichte Bevorzugung für Creme 1. Das Hautgefühl von Creme 1 wiederum wurde ganz stark dem von Creme 2 bevorzugt. Als Konsequenz daraus hat sich eine eindeutige Mehrheit der Probanden für Creme 1 entschieden.

Creme 1

| A | Polyethersiloxan gemäß Bezugsbeispiel 4 | 1,5 % |
|---|---|---|
| | Ceteareth-25 | 1,0 % |
| | Glyceryl Stearate | 2,5 % |
| | Stearyl Alcohol | 1,5 % |
| | Stearic Acid | 1,0 % |
| | Caprylic/Capric Triglyceride | 6,0 % |
| | Cetearyl Ethylhexanoate | 6,5 % |
| B | Glycerin | 2,0 % |
| | Water | 77,5 % |
| C | TEGO® Carbomer 134 (Carbomer) | 0,1% |
| | Paraffinum Liquidum | 0,4 % |

Creme 2

| A | Ceteareth-25 | 2,0 % |
|---|---|---|
| | Glyceryl Stearate | 2,5 % |
| | Stearyl Alcohol | 1,5 % |
| | Stearic Acid | 1,0 % |
| | Caprylic/Capric Triglyceride | 6,5 % |
| | Cetearyl Ethylhexanoate | 6,5 % |
| B | Glycerin | 2,0 % |
| | Water | 77,5 % |
| C | TEGO® Carbomer 134 (Carbomer) | 0,1% |
| | Paraffinum Liquidum | 0,4 % |

Beispiel 13:

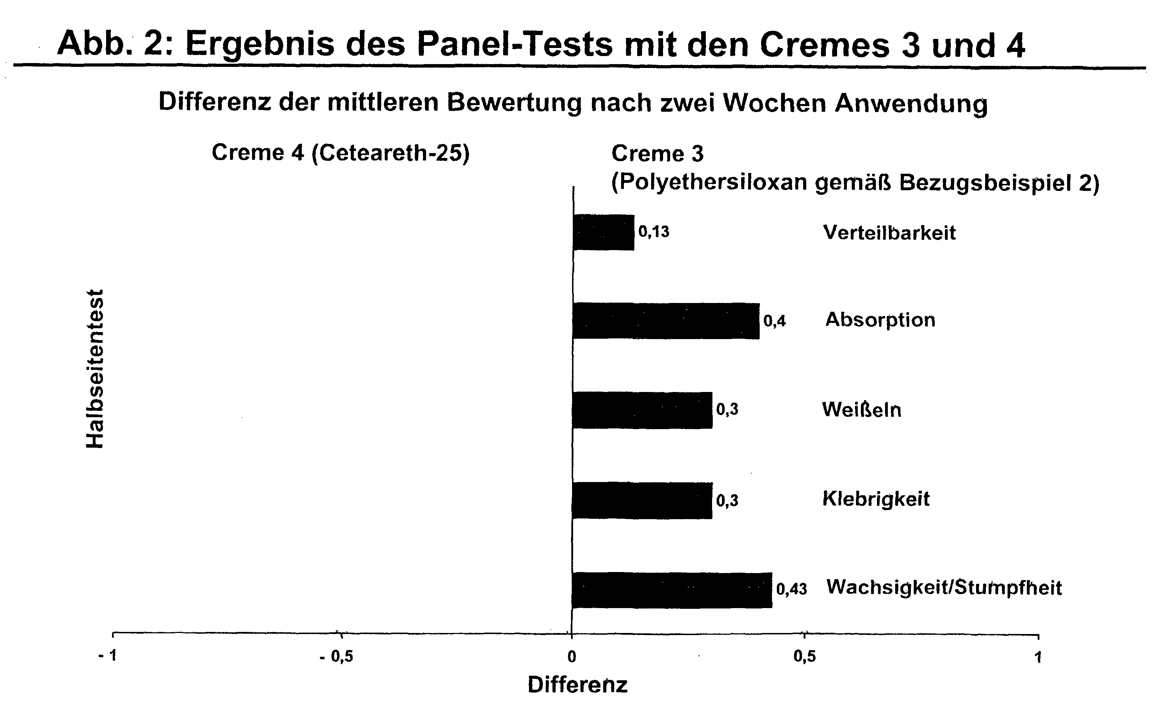

In einem Panel-Test wurden 20 Probanden gebeten, zwei Cremes direkt miteinander zu vergleichen. Creme 3 enthielt eine Kombination aus dem Polyethersiloxan gemäß Bezugsbeispiel 2 und Ceteareth-25 als Co-Emulgator, Creme 4 enthielt ausschließlich Ceteareth-25 als Emulgator (siehe Rezepturen Creme 3 und Creme 4). In Abb. 2 ist das Ergebnis des Paneltests dargestellt: Hinsichtlich der Attribute Verteilbarkeit, Absorption, Weißeln, Klebrigkeit und Wachsigkeit/Stumpfheit wurde Creme 3 signifikant der Creme 4 bevorzugt.

Creme 3

| A | Polyethersiloxan gemäß Bezugsbeispiel 2 | 1,% |
|---|---|---|

(fortgesetzt)

|   | Ceteareth-25 | 0,% |
|---|---|---|
|   | Glyceryl Stearate | 1,% |
|   | Stearyl Alcohol | 2,% |
|   | Stearic Acid | 1,% |
|   | Paraffinum Liquidum | 6,% |
|   | Ethylhexyl Stearate | 6,% |
| B | Glycerin | 3,% |
|   | Water | 77,% |

Creme 4

| A | Ceteareth-25 | 2,% |
|---|---|---|
|   | Glyceryl Stearate | 1,% |
|   | Stearyl Alcohol | 2,% |
|   | Stearic Acid | 1,% |
|   | Paraffinum Liquidum | 6,% |
|   | Ethylhexyl Stearate | 6,% |
| B | Glycerin | 3,% |
|   | Water | 77,% |

Beispiel 14:

Von einer Sonnenschutzlotion mit dem Polyethersiloxan gemäß Bezugsbeispiel 1 wurde in vivo gemäß Colipa die Wasserfestigkeit geprüft. Zu diesem Zweck wird der Lichtschutzfaktor bestimmt und die Messung nach Wässerung der behandelten Stelle wiederholt. Die Lotion hatte vor der Wässerung einen Lichtschutzfaktor von 14 und nach der Wässerung einen Lichtschutzfaktor von 10. Dies entspricht einer Wasserfestigkeit von 71 %. Ein Produkt kann als wasserfest bezeichnet werden, wenn die Wasserfestigkeit mindestens 50 % beträgt. Es ist besonders darauf hinzuweisen, dass die Rezeptur keine Inhaltsstoffe enthält, die ausdrücklich zur Erhöhung der Wasserfestigkeit eingesetzt werden wie z. B. filmbildende Polymere.

Sonnenschutzlotion

| A | Polyethersiloxan gemäß Bezugsbeispiel 1[1] | 1,7 % |
|---|---|---|
|   | ABIL® B 8863 | 0,3 % % |
|   | C12-15 Alkyl Benzoate | 3,0 % |
|   | Paraffinum Liquidum | 3,4 % |
|   | 4-Methylbenzylidene Camphor | 3,0 % |
|   | Ethylhexyl Methoxycinnamate | 2,5 % |
|   | Butyl Methoxydibenzoylmethane | 2,0 % |
|   | Isoamyl p-Methoxycinnamate | 2,5 % |

[1] ABIL® B 8863: Kammartiges Polyethersiloxan mit Gewichtsanteil der Polyetherreste an der Gesamtmolekularmasse von 76 %.

(fortgesetzt)

| | | | |
|---|---|---|---|
| | | Tocopheryl Acetate | 0,5 % |
| | B | TEGO® SMO 80 (Polysorbate 80) | 0,2 % |
| | | Glycerin | 2,0 % |
| | | EDTA | 0,1 % |
| | | GluCare® S (Sodium Carboxymethyl Betaglucan) | 0,1 % |
| | | Water | 75,9 % |
| | C | TEGO® Carbomer 140 (Carbomer) | 0,15% |
| | | TEGO® Carbomer 141 (Carbomer) | 0,15 % |
| | | Xanthan Gum | 0,1 % |
| | | Isopropyl Palmitate | 1,6 % |
| | D | Sodium Hydroxide (10 % in water) | 0,8 % |
| | | Konservierungsmittel, Parfum | q. s. |

**Patentansprüche**

1. Kosmetische oder pharmazeutische Öl-in-Wasser-Emulsionen, die ein oder mehrere Polyethersiloxane der allgemeinen Formel (I)

$$R(CH_3)_2SiO-[(CH_3)_2SiO]_n-Si(CH_3)_2R \qquad (I)$$

wobei
n = 50 bis 250

$$R = -(CH_2)_m-O-(C_2H_4O)_x-(C_3H_6O)_yR^1$$

m = 2 bis 4
x = 3 bis 100
y = 0 bis 50
$R^1$ = H, CH$_3$, oder CH$_2$CH$_3$
ist,
mit einem Gewichtsanteil der Polyetherreste R von bis zu 45 Gew. % an der Gesamtmolekularmasse, berechnet nach Formel (II)

"Gewichtsanteil (in %) der Polyetherreste R an der

Gesamtmolekularmasse" = $(MG_{Polyetherreste}/MG_{Gesamt})•100 \qquad (II)$

mit

$$MG_{Gesamt} = MG_{Siliconrest} + MG_{Polyetherreste}$$

$$MG_{Siliconrest} = n • 74,1 + 132,2$$

$$MG_{Polyetherreste} = 2 • (m • 14 + 16 + x • 44 + y • 58 + z)$$

mit z = 1, 15 oder 29
enthalten.

2. Kosmetische oder pharmazeutische Öl-in-Wasser-Emulsion nach Anspruch 1, enthaltend

    (a) ein oder mehrere Polyethersiloxane der allgemeinen Formel (I)
    (b) gegebenfalls einen oder mehrere Co-Emulgatoren.
    (c) flüssig-kristalline Strukturen ausbildende hydrophile Wachse und/oder wasserquellbare Organopolymere als Konsistenzgeber und Stabilisatoren,
    (d) kosmetische Öle und Wachse und
    (e) übliche Hilfs- und Wirkstoffe.

3. Kosmetische oder pharmazeutische Öl-in-Wasser-Emulsionen nach Anspruch 1 oder 2, **dadurch gekennzeichnet, daß** diese polare Wachse ausgewählt aus der Gruppe, bestehend aus Stearylalkohol, Stearinsäure und/oder Glycerylstearat als Konsistenzgeber und einen Co-Emulgator enthalten.

4. Kosmetische oder pharmazeutische Öl-in-Wasser-Emulsionen nach Anspruch 2 oder 3, **dadurch gekennzeichnet, daß** der Anteil des Polyethersiloxans der allgemeinen Formel (I) im Fall der Anwesenheit von Co-Emulgatoren, bezogen auf die Gesamtmenge der Emulgatoren wenigstens 50 Gew. %, insbesondere 65 bis 90 Gew. % beträgt.

5. Kosmetische oder pharmazeutische Öl-in Wasser-Emulsionen nach einem der Ansprüche 2 bis 4, **dadurch gekennzeichnet, daß** der Anteil des Co-Emulgators an der Gesamtmenge der Emulgatoren 5 bis 49 Gew.-% insbesondere 10 bis 35 Gew.-% beträgt.

6. Kosmetische oder pharmazeutische Öl-in-Wasser-Emulsionen nach einem der vorhergehenden Ansprüche **dadurch gekennzeichnet, dass** die weiteren Hilfs- und Zusatzstoffe ausgewählt sind aus der Gruppe bestehend aus UV-Lichtschutzfiltern, Antioxidantien, Konservierungsmittel, Insekten-Repellentien, Selbstbräunern, Parfümölen, Farbstoffen und Wirkstoffen.

7. Kosmetische oder pharmazeutische Öl-in-Wasser-Emulsionen nach einem der vorhergehenden Ansprüche, **dadurch gekennzeichnet, daß** sie frei von siliconartigen Ölkomponenten sind.

8. Kosmetische oder pharmazeutische Öl-in-Wasser-Emulsionen nach einem der vorhergehenden Ansprüche, **dadurch gekennzeichnet, daß** diese frei von kettenförmigen oder flüchtigen cylischen Polydimethylsiloxanen sind.

**Claims**

1. Cosmetic or pharmaceutical oil-in-water emulsions which comprise one or more polyether siloxanes of the general formula (I)

$$R(CH_3)_2SiO\text{-}[(CH_3)_2SiO]_n\text{-}Si(CH_3)_2R \qquad\qquad (I)$$

where
n = 50 to 250

$$R = \text{-}(CH_2)_m\text{-}O\text{-}(C_2H_4O)_x\text{-}(C_3H_6O)_yR^1$$

m = 2 to 4
x = 3 to 100
y = 0 to 50
$R^1$ = H, $CH_3$, or $CH_2CH_3$,
having a proportion by weight of the polyether radicals R of up to 45% by weight of the total molecular mass, calculated according to formula (II)

"proportion by weight" (in %) of the polyether radicals R

$$\text{of the total molecular mass"} = (MW_{\text{polyether radicals}}/MW_{\text{total}}) \bullet 100 \qquad (II)$$

where

$$MW_{\text{total}} = MW_{\text{silicone radical}} + MW_{\text{polyether radicals}}$$

$$MW_{\text{silicone radical}} = n \bullet 74.1 + 132.2$$

$$MW_{\text{polyether radicals}} = 2 \bullet (m \bullet 14 + 16 + x \bullet 44 + y \bullet 58 + z)$$

where z = 1, 15 or 29.

2. Cosmetic or pharmaceutical oil-in-water emulsion according to Claim 1, comprising

    (a) one or more polyether siloxanes of the general formula (I)
    (b) optionally one or more coemulsifiers
    (c) liquid-crystalline-structure-forming hydrophilic waxes and/or water-swellable organopolymers as bodying agents and stabilizers,
    (d) cosmetic oils and waxes and
    (e) customary auxiliaries and active ingredients.

3. Cosmetic or pharmaceutical oil-in-water emulsions according to Claim 1 or 2, **characterized in that** they comprise polar waxes chosen from the group consisting of stearyl alcohol, stearic acid and/or glyceryl stearate as bodying agents and a coemulsifier.

4. Cosmetic or pharmaceutical oil-in-water emulsions according to Claim 2 or 3, **characterized in that** the proportion of the polyether siloxane of the general formula (I) in the case of the presence of coemulsifiers is, based on the total amount of the emulsifiers, at least 50% by weight, in particular 65 to 90% by weight.

5. Cosmetic or pharmaceutical oil-in-water emulsions according to one of Claims 2 to 4, **characterized in that** the proportion of the coemulsifier of the total amount of the emulsifiers is 5 to 49% by weight, in particular 10 to 35% by weight.

6. Cosmetic or pharmaceutical oil-in-water emulsions according to one of the preceding claims, **characterized in that** the further auxiliaries and additives are chosen from the group consisting of UV light protection filters, antioxidants, preservatives, insect repellents, self-tanning agents, perfume oils, dyes and active ingredients.

7. Cosmetic or pharmaceutical oil-in-water emulsions according to one of the preceding claims, **characterized in that** they are free from silicone-like oil components.

8. Cosmetic or pharmaceutical oil-in-water emulsions according to one of the preceding claims, **characterized in that** they are free from chain-shaped or volatile cyclic polydimethylsiloxanes.

**Revendications**

1. Emulsions huile-dans-eau cosmétiques ou pharmaceutiques, qui contiennent un ou plusieurs polyéthersiloxanes de formule générale (I)

$$R(CH_3)_2SiO\text{-}[(CH_3)_2SiO]_n\text{-}Si(CH_3)_2R \qquad (I)$$

où
n = 50 à 250

$$R = -(CH_2)_m-O-(C_2H_4O)_x-(C_3H_6O)_yR^1$$

m = 2 à 4
x = 3 à 100
y = 0 à 50
$R^1 = H, CH_3$ ou $CH_2CH_3$,
avec une proportion pondérale des radicaux polyéther R allant jusqu'à 45% en poids par rapport à la masse moléculaire totale, calculée selon la formule (II)

$$\text{"Proportion pondérale (en \%) des radicaux}$$

$$\text{polyéther R par rapport à la masse moléculaire}$$

$$\text{totale"} = (PM_{radical\ polyéther}/PM_{total}) \cdot 100 \qquad (II)$$

où

$$PM_{total} = PM_{radical\ silicone} + PM_{radical\ polyéther}$$

$$PM_{radical\ silicone} = n \cdot 74,1 + 132,2$$

$$PM_{radical\ polyéther} = 2 \cdot (m \cdot 14 + 16 + x \cdot 44 + y \cdot 58 + z)$$

avec z = 1, 15 ou 29.

**2.** Emulsion huile-dans-eau cosmétique ou pharmaceutique selon la revendication 1, contenant

    (a) un ou plusieurs polyéthersiloxanes de formule générale (I),
    (b) le cas échéant un ou plusieurs co-émulsifiants,
    (c) des cires hydrophiles formant des structures liquides-cristallines et/ou des organopolymères qui gonflent dans l'eau comme agents conférant de la consistance et stabilisateurs,
    (d) des huiles et des cires cosmétiques et
    (e) des adjuvants usuels et des substances actives usuelles.

**3.** Emulsions huile-dans-eau cosmétiques ou pharmaceutiques selon la revendication 1 ou 2, **caractérisées en ce qu'**elles contiennent des cires polaires choisies dans le groupe constitué par l'alcool stéarylique, l'acide stéarylique et/ou le stéarate de glycéryle comme agent conférant de la consistance et un co-émulsifiant.

**4.** Emulsions huile-dans-eau cosmétiques ou pharmaceutiques selon la revendication 2 ou 3, **caractérisées en ce que** la proportion de polyéthersiloxane de formule générale (I), en présence de co-émulsifiants, par rapport à la quantité totale des émulsifiants, est d'au moins 50% en poids, en particulier de 65 à 90% en poids.

**5.** Emulsions huile-dans-eau cosmétiques ou pharmaceutiques selon l'une quelconque des revendications 2 à 4, **caractérisées en ce que** la proportion de co-émulsifiant par rapport à la quantité totale des émulsifiants est de 5 à 49% en poids, en particulier de 10 à 35% en poids.

**6.** Emulsions huile-dans-eau cosmétiques ou pharmaceutiques selon l'une quelconque des revendications précédentes, **caractérisées en ce que** les autres adjuvants et additifs sont choisis dans le groupe constitué par les filtres de protection contre la lumière UV, les antioxydants, les conservateurs, les agents répulsifs d'insectes, les auto-bronzants, les huiles parfumées, les colorants et les substances actives.

7. Emulsions huile-dans-eau cosmétiques ou pharmaceutiques selon l'une quelconque des revendications précédentes, **caractérisées en ce qu'**elles sont exemptes de composants huileux de type silicone.

8. Emulsions huile-dans-eau cosmétiques ou pharmaceutiques selon l'une quelconque des revendications précédentes, **caractérisées en ce qu'**elles sont exemptes de polydiméthylsiloxanes linéaires ou cycliques volatils.

# Abb. 1: Ergebnis des Panel-Tests mit den Cremes 1 und 2

EP 1 125 574 B1

# Abb. 2: Ergebnis des Panel-Tests mit den Cremes 3 und 4

**Differenz der mittleren Bewertung nach zwei Wochen Anwendung**

**Creme 4 (Ceteareth-25)**          **Creme 3
(Polyethersiloxan gemäß Bezugsbeispiel 2)**

| | |
|---|---|
| 0,13 | Verteilbarkeit |
| 0,4 | Absorption |
| 0,3 | Weißeln |
| 0,3 | Klebrigkeit |
| 0,43 | Wachsigkeit/Stumpfheit |

Halbseitentest

- 1          - 0,5          0          0,5          1

**Differenz**

EP 1 125 574 B1